# EUROPEAN PATENT APPLICATION

(11) **EP 2 130 905 A1**
(43) Date of publication of application: **09.12.2009**
(21) Application number: 08157551.6
(22) Date of filing: 04.06.2008
(51) Int. Cl.: C12M 3/06

(54) **Method for culturing eukaryotic cells**

(71) Applicant: Pharmacell B.V., 6229 EV Maastricht (NL)
(72) Inventor: Lardenoije, René Joseph Gerardus Marie, 2314 EP Leiden (NL)
(74) Representative: Habets, Winand

(57) **Abstract**

The invention is in the field of biotechnology and production of useful biomolecules. It relates to a method for culturing eukaryotic cells, more in particular adherent cells. The method is useful for the *in vitro* generation of tissue, for the production of biomolecules such as proteins and for use in regenerative medicine. The invention provides a bioreactor system comprising a membrane and a matrix suitable for cells to adhere to. The invention provides a bioreactor suitable for culturing adherent cells in a culture medium wherein said bioreactor comprises an internal chamber (11) and an external chamber (12) separated by a selective permeable membrane (22), the internal chamber comprising a three dimensional matrix (23) suitable for supporting the adherence of said cells, said matrix being surrounded by the selective permeable membrane, wherein the bioreactor comprises a feed medium inlet (31) and a feed medium outlet (32), both connected to the internal chamber and a spent medium inlet (41) and spent medium outlet (42), both connected to the external chamber.

## Description

### Field of the invention

The invention is in the field of biotechnology and production of useful biomolecules. It relates to a method for culturing eukaryotic cells, more in particular adherent cells. The method is useful for the *in vitro* generation of tissue, for the production of biomolecules such as proteins and for use in regenerative medicine. The invention provides a bioreactor system comprising a membrane and a matrix suitable for cells to adhere to.

### Background of the invention

Methods for cell culturing are known. Cell culture is the process by which prokaryotic, eukaryotic or plant cells are grown under controlled conditions. In practice the term "cell culture" has come to refer to the culturing of cells derived from multi-cellular eukaryotes, especially animal cells. The historical development and methods of cell culture are closely interrelated to those of tissue culture and organ culture.

Animal cell culture became a routine laboratory technique in the 1950s but the concept of maintaining live cell lines separated from their original tissue source was already discovered in the 19th century (reviewed in: Molecular biology of the cell, Bruce Alberts et al. 4th edition ISBN 0-8153-3218-1)

Cells are grown and maintained at an appropriate temperature and gas mixture (typically, 37°C, 5% CO2) in a cell incubator. Culture conditions vary widely for each cell type, and variation of conditions for a particular cell type can result in different phenotypes being expressed.

Aside from temperature and gas mixture, the most commonly varied factor in culture systems is the growth medium. Recipes for growth media can vary in pH, glucose concentration, growth factors, and the presence of other nutrient components. The growth factors used to supplement media are often derived from animal blood, such as calf serum. These blood-derived ingredients pose the potential for contamination of derived pharmaceutical products with viruses or prions. Current practice is to minimize or eliminate the use of these ingredients where possible.

Some cells naturally live without attaching to a surface, such as cells that exist in the bloodstream. Others require a surface, such as most cells derived from solid tissues. Cells grown unattached to a surface are referred to as suspension cultures.

Cells can be grown in suspension or adherent cultures. Some cells naturally live in suspension, without being attaching to a surface, such as cells that exist in the bloodstream. There are also cell lines that have been modified to be able to survive in suspension cultures so that they can be grown to a higher density than adherent conditions would allow. Adherent cells require a surface, such as tissue culture plastic, which may be coated with extracellular matrix components to increase adhesion properties and provide other signals needed for growth and differentiation. Most cells derived from solid tissues are adherent.

Another type of adherent culture is organotypic culture which involves growing cells in a three-dimensional environment, as opposed to two-dimensional culture dishes. The 3-dimensional environment suitable for attachment and supporting growth of adherent cells is often referred to as a matrix or a scaffold or a substrate. This 3D culture system is biochemically and physiologically more similar to in vivo tissue, but is technically challenging to maintain because of many factors. One of the most challenging problems to overcome in 3D culture system is the supply of nutrients and other essential components to the cells throughout the matrix. This process is often limited by the diffusion rates of the components involved.

As cells generally continue to divide in culture, they generally grow to fill the available area or volume. This can generate several issues such as nutrient depletion in the growth media and accumulation of apoptotic/necrotic (dead) cells. Furthermore, cell-to-cell contact can stimulate cell cycle arrest, causing cells to stop dividing. This phenomenon is known as contact inhibition. Also, cell-to-cell contact can stimulate promiscuous and unwanted cellular differentiation.

The purpose of media changes is to replenish nutrients and avoid the build up of potentially harmful metabolic byproducts and dead cells. In the case of suspension cultures, cells can be separated from the media by centrifugation and resuspended in fresh media. In the case of adherent cultures, the media can be removed directly by aspiration and replaced.

US2003036192 provides a disposable bioreactor for perfusion cell culture. Cells are therein grown in a plastic bag that is rocked and aerated on a mechanical platform, thereby preventing the filter from clogging.

A typical way of culturing cells in a 3-D culture system employs matrices such as a collagen matrix capable of providing support to adherent cells. Traditional bioreactors are designed as stationary pressurized vessels. Current bioreactors are disposable devices which commonly utilize plastic sterile bags. A typical example of such a disposable culture system is provided in EP1132460 which discloses a method for culturing dendritic cells in a disposable culture bag comprising a matrix that supports the growth of dendritic cells.

Certain cells or tissue can only be cultured well if a certain shear flow stress is applied. US5928945 discloses the culturing of chondrocytes in a three-dimensional matrix in a disposable culture vessel under appropriate shear flow stress. EP1077072 discloses the culturing of artificial homologues heart valves and blood vessels in a culture vessel that provide a pulsating flow of culture medium.

One particular disadvantage of the prior art methods that employ collagen matrices is that only very thin layers of cells may be achieved because nutrients become depleted very soon when the thickness of a layer increases beyond a critical value.

WO 2006/023136 addresses this problem and provides a method of generating tissue from stem and progenitor cells wherein primary mammalian stem cells and progenitor cells are placed in an extracellular matrix and the matrix is maintained in a culture medium in a microgravity environment. This method suffers from the shortcoming that it is expensive and technically challenging to operate and maintain. It is hardly scalable and does not allow the use of disposable culture chambers.

US2005/0272146 is also concerned with this problem and provides the solution to include a disposable mixing device in the culture system.

There remains an obvious need in the art for efficient and cheap bioreactors that allow for the culturing of adherent cells in a three-dimensional matrix.

### Summary of the invention

The invention provides a bioreactor suitable for culturing adherent cells in a culture medium wherein said bioreactor comprises an internal chamber (11) and an external chamber (12) separated by a selective permeable membrane (22), the internal chamber comprising a three dimensional matrix (23) suitable for supporting the adherence of said cells, said matrix being surrounded by the selective permeable membrane, wherein the bioreactor comprises a feed medium inlet (31) and a feed medium outlet (32), both connected to the internal chamber and a spent medium inlet (41) and spent medium outlet (42), both connected to the external chamber.

The invention further provides a method for culturing adherent cells in a culture medium wherein said cells are seeded into a three dimensional matrix suitable for supporting the adherence of said cells and incubated under growth-supporting conditions in a bioreactor described above, **characterized in that** a flow of culture medium is maintained in at least two distinct directions, a first direction essentially parallel to the surface of the matrix and a second direction essentially through the matrix.

The invention also provides tissue produced by the method described above.

### Detailed description of the invention

The invention provides a bioreactor suitable for culturing adherent cells in a culture medium wherein said bioreactor comprises an internal chamber (11) and an external chamber (12) separated by a selective permeable membrane (22), the internal chamber comprising a three dimensional matrix (23) suitable for supporting the adherence of said cells, said matrix being surrounded by the selective permeable membrane, wherein the bioreactor comprises a feed medium inlet (31) and a feed medium outlet (32), both connected to the internal chamber and a spent medium inlet (41) and spent medium outlet (42), both connected to the external chamber.

As used herein, a "three dimensional matrix" means a porous, cell culture-compatible supporting structure to which cultured cells can anchor or attach, when maintained under appropriate conditions in a growth chamber. This is also often referred to as "substrate" or "scaffold"

In order to provide a controlled flux of nutrients across the matrix, a culture chamber is designed comprising an internal chamber surrounded by a selective permeable membrane.

A selective permeable membrane is a membrane that allows the selective passage of molecules based on charge, size or other distinguishing features. In a preferred embodiment, the membrane may be attached to a structural support (21) for further structural support.

Alternatively or additionally, the matrix may also be confined by the selective permeable membrane and/or be attached, for instance chemically or physically to the membrane, in part or in its entirety, to provide further structural support.

The invention therefore relates to a bioreactor as described above wherein the membrane (22) and/or the matrix (23) are supported by a support (21). The support 21 may be a separate entity or be that the membrane itself supports the matrix. Providing a separate support has the advantage that the shape of the matrix can be pre-determined. If the shape of the matrix is irrelevant, the bioreactor may operate without a solid support. The matrix may in that case exhibit an amorphous shape.

The three-dimensional matrices or scaffolds in the bioreactor according to the invention may be made from polymer material or from a natural origin such as collagen.

Within the internal chamber, a biocompatible, porous matrix or scaffold, such as polymerized collagen may be positioned. The porous matrix is preferably suitable for cells to adhere to and proliferate in. If a tissue is to be produced, the matrix or scaffold has the shape of the required engineered tissue.

The invention also relates to a bioreactor as described above, further equipped with appliances that allow a flow of culture medium to be applied in the bioreactor in at least two distinct directions, a first direction essentially parallel to the surface of the matrix and a second direction essentially through the matrix.

The internal chamber may be situated into an external chamber. By applying a specified pressure differential between the internal and the external chamber a predefined flux of nutrient medium can be realized through the matrix providing sufficient nutrients to all cells growing in the matrix and, therefore avoiding necrotic cores in the cultured tissue.

Fluid connections attached to the internal and/or external chambers may be made of polymeric tubing suitable for sterile welding connections. The internal and/or external chambers may contain a monitor device and a sensor to control parameters such as pH, dissolved oxygen and temperature.

The bioreactor according to the invention may therefore be equipped with control probes (20) that measure and control parameters selected from the group consisting of pH, temperature, dissolved oxygen, Pi, Pe and flow rates.

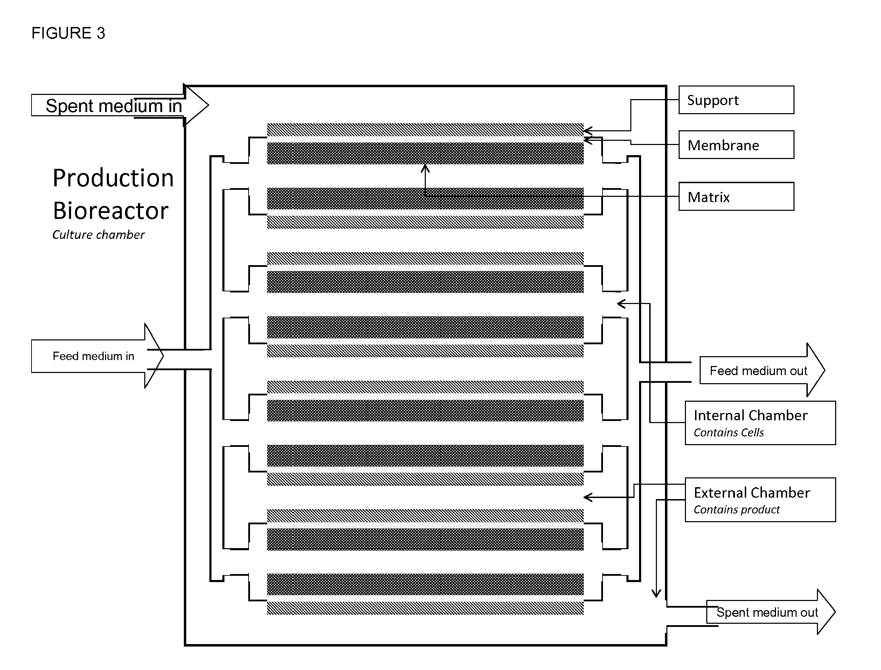

The internal chamber may be designed as a disposable cassette comprising at least a three dimensional matrix and a selectively permeable membrane. The bioreactor, the control and the feeding system may then be designed as a closed system capable of receiving the matrix/membrane cassette so that the bioreactor may be easily applied in less qualified environments. Advantageously, the bioreactor is designed to receive a multitude of matrix/membrane cassettes. An example of such a reactor is shown in figure 3.

The bioreactor according to the invention is particularly useful for culturing cells for a clinical application. In a preferred embodiment, the internal chamber wherein the cells are in contact with the feed medium is designed as a disposable compartment whereas the external chamber is designed as a multi-use compartment. The use of a disposable internal chamber avoids cross contamination of individual cell samples.

The matrix/membrane cassette may be enclosed by a biocompatible polymeric (plastic) structure such as a bag or a chamber.

Electrical connections and sensors may be applied through conventional means known in the art.

The bioreactor may be equipped with a device for exchanging gasses with the feed medium. The bioreactor may also be equipped with biosensors for monitoring biological active components. It is also advantageous to apply a gas exchange chamber capable of providing sufficient oxygen to the feed stream medium.

In a further embodiment, the invention provides a method that allows for the preparation of high density adherent cell cultures on an extracellular matrix when an efficient nutrient flow is established across the matrix. The invention therefore provides a method for culturing adherent cells in a culture medium wherein said cells are seeded into a three dimensional matrix suitable for supporting the adherence of said cells and incubated under growth-supporting conditions in a bioreactor as described above, **characterized in that** a flow of culture medium is maintained in at least two distinct directions, a first direction essentially parallel to the surface of the matrix and a second direction through the matrix.

By applying a specified pressure gradient between the internal and the external chamber a predefined flux of nutrient medium can be realized through the matrix providing sufficient nutrients to all cells growing in the matrix and, therefore avoiding necrotic cores in the cultured tissue.

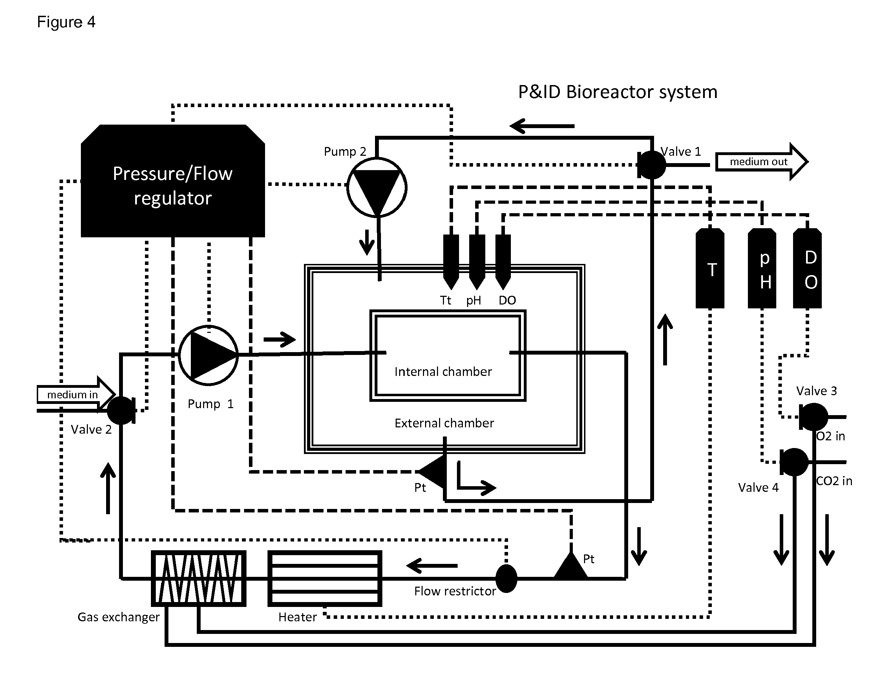

In order to apply a pressure gradient over the internal and external chambers, two distinct circuits of fluid flow may be created. First, recirculation of feed medium through the internal chamber may be controlled with one pump whereas circulation of spent medium in the outer compartment may be controlled by another pump (see figures 3 and 4). Alternatively, one single pump may be used to create both flows, the pressure gradient between internal and external chambers then has to be created by other means.

A pressure gradient may also be established by a difference in osmotic pressure between the internal and external chambers. The term "pressure gradient" in this context is to be understood as any force capable of driving a liquid flow across the matrix and/or the membrane.

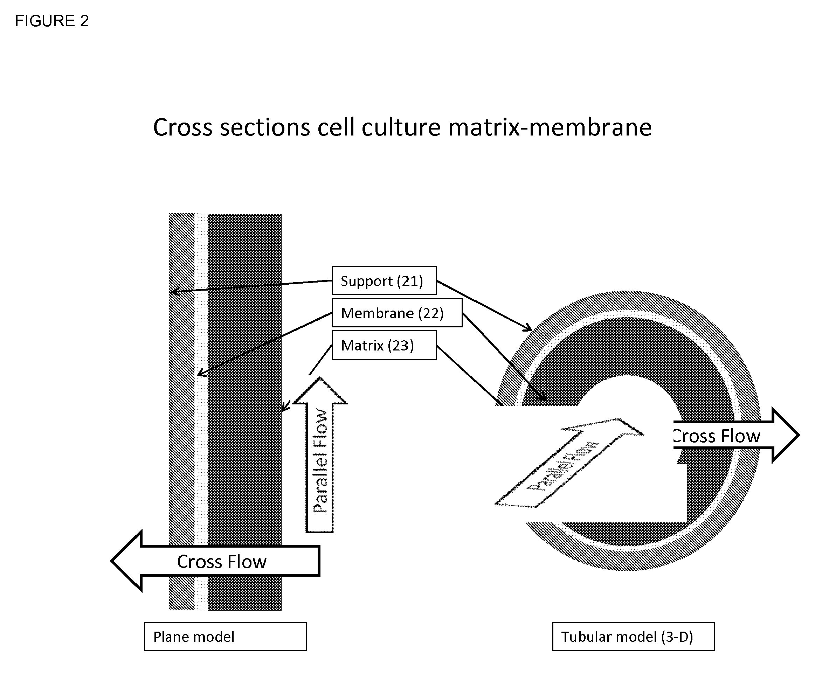

Figure 2 depicts the interpretation of the terms "essentially parallel" and "through" the matrix. A cross-flow is to be interpreted as a flow through the matrix, preferably essentially perpendicular to the length of the matrix, whereas a parallel flow is to be interpreted as a flow essentially in the direction of the length of the matrix. A person skilled in the art will be aware of the proper interpretation of these terms.

The method of the invention is particularly suited for culturing proliferative cells. Therefore the invention relates to a method as described above wherein the adherent cells are proliferative cells.

The method may advantageously be employed to culture cells that produce and excrete certain biological molecules. The selective permeable membrane can be chosen in such a way that it is permeable for this product and, therefore, the product can be harvested from the external fluid circuit, i.e. the external chamber.

Preferably, the membrane is semi-permeable to allow efficient harvesting of cell-excretions from the spent medium in the external compartment. The invention therefore relates to a method as described above wherein the membrane is semi-permeable. If the cell excretion product is a protein, then the membrane is preferably semi-permeable for proteins of a certain molecular weight.

The invention also relates to a method as described above wherein the membrane is endorsed by a solid support, such as a polymeric support. This has the advantage that the shape of the matrix can be pre-determined. If the shape of the matrix is irrelevant, the bioreactor may operate without a solid support of the membrane. The matrix may in that case exhibit an amorphous shape.

Cells may be seeded into the matrix by adding a cell suspension to the feed medium. Because of the combined parallel flow and cross flow through the membrane, the cells will be homogenously spread over the matrix. The invention therefore relates to a method as described above wherein the cells are seeded into the three dimensional matrix by means of addition of a cell suspension to the flow of culture medium, causing a homogenous influx of cells into the matrix. This seeding regimen has the large advantage or minimizing the number of open handlings, thus reducing the risk of contamination. The seeding process is preferably conducted while the matrix is present in the internal chamber in the bioreactor.

A particularly advantageous way to maintain a suitable flow of culture medium was found to be the application of hydrodynamic flow conditions.

The method according to the invention is particularly suited for the culturing of progenitor cells. It may also be used for the generation of a tissue such as smooth muscle tissue or cardiac muscle tissue, nerve tissue, cartilage, bone, dermis or epidermis. Cells used in the method according to the invention may be selected from the group consisting of transgenic, immortalized cells and tumor cell lines. The cells may also be stable transfected human primary cells.

Adherent cells such as stem or progenitor cells may either be obtained from a donor or available as an established cell line.

Additionally, the invention provides a method wherein the cells are capable of expressing and/or secreting a recombinant protein. The cell excretion product may then be harvested from the spent medium in the external chamber.

In an advantageous embodiment of the invention, the matrix comprises type 1 collagen, such as type-1 human collagen or wherein the collagen is of recombinant origin.

The methods according to the invention allow for the engineering of living tissue in vitro. They may also provide an efficient way of producing large amounts of adherent (progenitor) cells for application in cell therapy.

The method according to the invention may also be used to achieve an engineered living tissue construct with an architecture specific for a particular tissue.

The method according to the invention may also be used to culture non-differentiated adherent progenitor cells in high cell numbers for application in cell therapy.

The flow of culture medium through the matrix (cross flow, figure 2) can mimic physiological properties required for optimal culturing of the engineered tissue, for example, as required for culturing blood vessels in vitro. Because of the two distinct flow directions applied in the method according to the invention, the bioreactor may also mimic a physiological function of life tissue, such as the function of a renal glomerular basement membrane.

For production of large amount of single cells, not organized as a tissue, the matrix surrounded by the membrane may have a rectangular shape, thus forming a cassette that may be multiplied in a bioreactor such that a scalable system may be obtained (Figure 3).

Addition of for example trypsin inhibitor and certain grow stimulating factors such as IGF, FGF, TGF-β may further stimulate the cells to adhere to the matrix and to proliferate.

Cell growth and/ or differentiation can be influenced by adding growth factors to the medium, to enhance the specificity of each seeded layer/ cell type. Mechanical forces can be put on the construct by applying a pulse pressure through the tubular structure in order to enhance extra cellular matrix production and expression of cytoskeletal protein in the cells in the construct. Thus, structural integrity will be improved.

The bioreactor and method according to the invention now allow for the production of multilayer tissue. The invention therefore also relates to tissue produced by any method as disclosed herein

### Legend to the figures

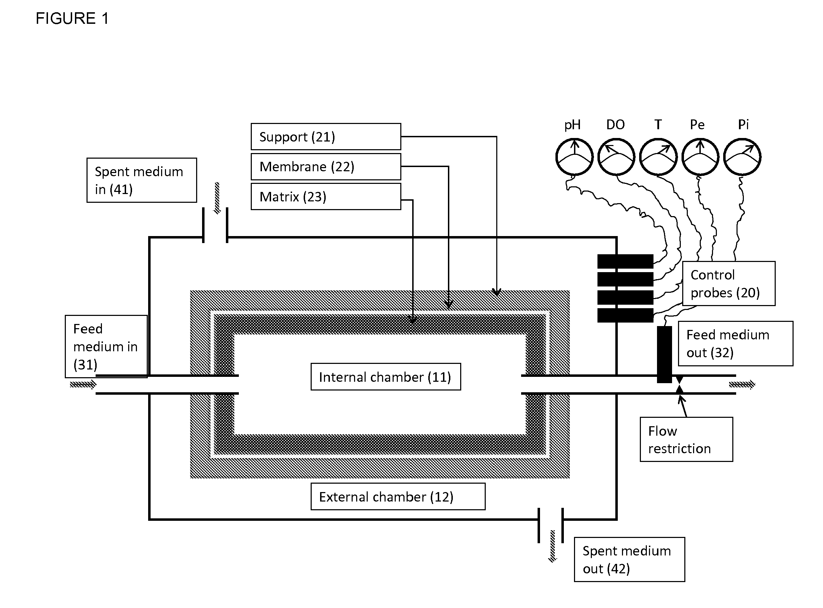

Figure 1: Overview of a bioreactor comprising a single cell culture chamber.
Figure 2: Cross-sections of cell culture matrix - membrane cassettes
Figure 3: Production bioreactor comprising multiple units of matrix-membrane cassettes
Figure 4: Design scheme (piping and Instrumentation Diagram (P&ID)) of a bioreactor according to the invention.

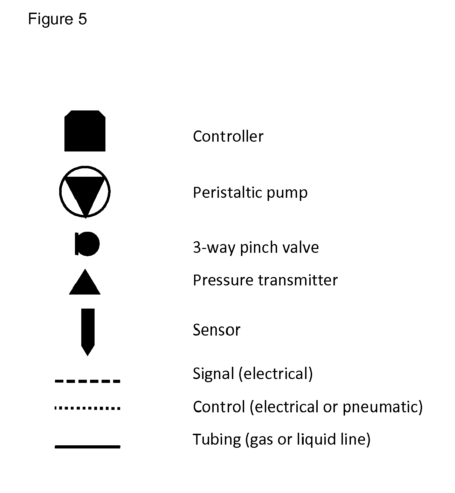

Figure 5: Explanation of symbols used in figure 4.

### Examples

### Example 1: Cell production unit

A bioreactor was produced for culturing of single porcine fibroblasts as a proof of principle. This unit comprised a regulatory unit for flow, temperature, feed medium (DMEM-HG with 10% FBS), gas concentration and spent medium control. It housed a cassette with closely packed laminea of a collagen matrix consisting of porcine type I collagen and elastin (Matricel GmbH, product # 3D001315) and a selective permeable membrane (Cellu-Sep T1, 5030-46) essentially as shown in figure 3. When a pressure differential was applied between the flow in the internal chamber and the external chamber, this caused a cross-flow through the matrix and membrane.

Cells were added to the feed medium and seeded into the matrix of the laminea by the cross-flow. Cells adhered to the matrix. After seeding and adherence, the flow through the matrix, over the membrane ensured that sufficient nutrients and oxygen are available for the cells to grow. Depending on matrix thickness, pressure differential was regulated to ensure sufficient flow through the membrane. As the cell numbers in the matrix increased, resistance increased as well. To avoid insufficient nutrient flow towards the cells, pressure differential was therefore adapted.

When a pre-defined threshold level in pressure was reached, confluence of the culture in the matrix was reached, and cells were harvested. Cell harvest was done by adding collagenase which broke down the matrix and released the cells. Alternatively, trypsin may be added to the medium to disconnect the cells from the matrix. Cells were harvested from the feed medium coming out of the cassette. In this way, the system allowed for an efficient, labor extensive, minimal exposed expansion culture of cells.

### Example 2: Tissue production unit

When used as a production unit for e.g. replacement tissue, the bioreactor comprised a regulatory unit for flow, temperature, feed medium, gas concentration and spent medium control. It housed a cassette with a matrix shaped in the form of a vascular graft.

A tubular shaped matrix from porcine type I collagen and elastin (Matricel GmbH) was surrounded by the selectively permeable membrane (Cellu-Sep T1, 5030-46). When a pressure differential was applied between the flow in the internal chamber and the external chamber, this caused a cross-flow through the matrix and membrane.

Procine fibroblasts, smooth muscle cells and endothelial cells were sequentially added to the medium flowing through the tube in order to achieve a layered structure of the construct. Fibroblast were added first, to form the adventitia, then smooth muscle cells were added to form a media, and finally endothelial cell were added to form an intima. Loading of the cells was separated in time to ensure grow of each layer before seeding the next.

When the smooth muscle cells were seeded and had time to attach, a pulsatile flow was applied through the construct to mimic the physiological stresses of the arterial flow. A pulsatile flow stimulates the production of both intercellular (smooth muscle actin, and myosine) as well as extracellular (Collagen type II and elastin) proteins that improve compliance and the mechanic strength of the product.

When endothelial cells were loaded, the medium was enriched using heparin TGF-β to reduce the de-differentiation of the seeded endothelial cells.

Once the pressure difference over the membrane reached a pre-defined threshold, tissue formation was completed and the construct was harvested from the device.

## Claims

1. A bioreactor suitable for culturing adherent cells in a culture medium wherein said bioreactor comprises an internal chamber (11) and an external chamber (12) separated by a selective permeable membrane (22), the internal chamber comprising a three dimensional matrix (23) suitable for supporting the adherence of said cells, said matrix being surrounded by the selective permeable membrane, wherein the bioreactor comprises a feed medium inlet (31) and a feed medium outlet (32), both connected to the internal chamber and a spent medium inlet (41) and spent medium outlet (42), both connected to the external chamber.

2. A bioreactor according to claim 1 wherein the three dimensional matrix is attached to the selective permeable membrane.

3. Bioreactor according to claims 1 or 2 wherein the membrane and/or the matrix are supported by a support (21).

4. Bioreactor according to claims 1 to 3 equipped with
a. at least one control probe (20) that measure and control parameters selected from the group consisting of pH, temperature, dissolved oxygen, Pe, Pi and flow rates and/or
b. at least one device for exchanging gasses with the feed medium and/or
c. at least one biosensor for monitoring the production of biological active components.

5. Method for culturing adherent cells in a culture medium wherein said cells are seeded into a three dimensional matrix suitable for supporting the adherence of said cells and incubated under growth-supporting conditions in a bioreactor according to claims 1 to 4, **characterized in that** a flow of culture medium is maintained in at least two distinct directions, a first direction essentially parallel to the surface of the matrix and a second direction through the matrix.

6. A method according to claim 5 wherein the adherent cells are proliferative cells.

7. A method according to claims 5 or 6 wherein the membrane is semi-permeable, preferably for biomolecules such as proteins of a certain molecular weight.

8. A method according to claims 5 to 7 wherein the membrane is endorsed by a solid support, such as a polymeric support.

9. A method according to claims 5 to 8 wherein the flow of culture medium is maintained by applying hydrodynamic flow conditions.

10. A method according to claims 5 to 9 wherein the cells are
a. progenitor cells, and/or
b. transgenic or immortalized cells or tumor cell lines and/or
c. capable of expressing and/or secreting a recombinant protein and/or
d. stable transfected human primary cells.

11. A method according to claims 5 to 10 for the generation of a tissue such as smooth muscle tissue or cardiac muscle tissue, nerve tissue, cartilage, bone, dermis or epidermis.

12. A method according to claims 5 to 11 wherein a cell excretion product such as a protein is harvested from the external chamber.

13. A method according to claims 5 to 12 wherein the matrix comprises type 1 collagen, preferably type-1 human collagen.

14. A method according to claim 13 wherein the collagen is of recombinant origin.

15. Tissue produced by any method according to claims 5 to 14.
